# EUROPÄISCHE PATENTSCHRIFT

(11) **EP 2 914 709 B1**
(45) Veröffentlichungstag und Bekanntmachung des Hinweises auf die Patenterteilung: **31.03.2021**
(21) Anmeldenummer: 13796010.0
(22) Anmeldetag: 30.10.2013
(51) Int. Cl.: C12M 1/107, B01F 15/00, C12M 1/00, C12M 1/06

(54) **FERMENTER EINER BIOGASANLAGE**
FERMENTER OF A BIOGAS SYSTEM
FERMENTEUR D'UNE INSTALLATION DE BIOGAZ

(30) Priorität: 30.10.2012 DE 102012021206
(43) Veröffentlichungstag der Anmeldung: 09.09.2015
(73) Patentinhaber: Uts Biogastechnik GmbH, 85399 Hallbergmoos (DE)
(72) Erfinder: CZWALUK, Andreas, 49377 Vechta (DE); BIERER, Johann, 84405 Dorfen (DE)
(74) Vertreter: BSB Patentanwälte Schütte & Engelen Partnerschaft mbB
(86) Internationale Anmeldenummer: PCT/EP2013/072745
(87) Internationale Veröffentlichungsnummer: WO 2014/068016

(56) Entgegenhaltungen:
- EP-A1- 2 270 128
- EP-B1- 1 717 305
- WO-A1-2011/082787
- WO-A2-2007/110775
- DE-A1- 3 420 094
- DE-U1-202009 005 024
- US-A1- 2012 122 200

## Beschreibung

Die vorliegende Erfindung betrifft einen Fermenter einer Biogasanlage mit wenigstens einer Fermenterwandung und wenigstens einem Fermenterinnenraum und einem Fermenterdach. In dem Innenraum des Fermenters ist eine Rühreinrichtung angeordnet, um das in dem Fermenterinnenraum vorhandene Substrat umzurühren. Die Rühreinrichtung ist an einer insbesondere senkrecht angeordneten Führungsstange als Führungseinheit verstellbar angeordnet.

Im Stand der Technik sind verschiedenste Biogasanlagen und Fermenter bekannt geworden. Das in dem Fermenter vorhandene Substrat wird in der Regel umgerührt, um eine Homogenisierung des Substrates zu erzielen und um ein Absetzen verschiedener Bestandteile zu verhindern. Im Betrieb muss regelmäßig die Ausrichtung der Rühreinrichtung verstellt werden, um Toträume zu durchspülen und für eine gute Durchmischung zu sorgen. Eine solche Neuausrichtung der Rühreinrichtung bzw. des Rührgerätes kann in regelmäßigen Abständen z. B. in 10 oder 14 Tagen erfolgen, wobei der zeitliche Abstand von dem Substrat und den weiteren äußeren Bedingungen abhängt. Die zum Umrühren eingesetzten Rührgeräte sind meist als Tauchrührgeräte ausgeführt und weisen in vielen Fällen, 2, 3 oder 4 Rührflügel auf. Das Rührgerät und insbesondere dessen Rührflügel unterliegen einem Verschleiß und müssen deshalb regelmäßig gewartet werden. Bei einer solchen Wartung können z.B. die Rührflügel einzeln oder insgesamt getauscht werden.

Um die Wartung zu erleichtern und um zu starkes Absenken des Füllstandes in dem Fermenter für die Wartung zu vermeiden, sind solche Rührgeräte in der Regel höhenverstellbar in dem Fermenter aufgenommen. Für die Wartung wird das Rührgerät nach oben verfahren, sodass das Rührgerät bzw. dessen Rührflügel besser zugänglich sind.

Um die Wartung und auch die Höhenverstellung sowie die Winkeleinstellung des Rührgerätes zu erleichtern, ist mit der EP 1 717 305 B1 ist Serviceschacht für einen Fermenterbehälter einer Biogasanlage bekannt geworden, welcher das flexibel vorgesehene Foliendach gasdicht durchstößt. Das Rührgerät wird an einer vertikalen Stützstange gehalten, die sich von dem Fermenterboden aus bis in den Serviceschacht hinein erstreckt. Zu Wartungszwecken kann das Rührgerät bis in den Wartungsschacht hinein nach oben verfahren werden, sodass das Rührgerät nach Öffnung der Türen des Serviceschachtes für Wartungszwecke bequem zur Verfügung steht.

Auch in der US 2012/12200 A1 wird ein Fermenter mit einem Serviceschacht beschrieben, wobei ein an einer Montagestage angeordnetes Rührgerät mittels einer auf dem Serviceschacht angeordneten Kurbel höhenverstellt werden kann.

Mit der DE 19 732 198 C1 ist eine Hubvorrichtung für ein Tauchmotorrührgerät eines Fermenterbehälters einer Biogasanlage bekannt geworden, wobei der mit einer Betondecke versehene Fermenterbehälter eine ausreichend große Öffnung aufweist, um das Tauchmotorrührgerät aus dem Fermenterbehälter hinaus in einen domartigen Behälter verfahren zu können. Dort ist das Tauchmotorrührgerät nach Öffnung des Domes für Wartungszwecke bequem zugänglich. Die Höhenverstellung des Tauchmotorrührgerätes verfolgt über einen Seiltrommelantrieb, wobei die Seiltrommel innerhalb des Grenzbereiches angeordnet ist und über einen elektrischen Motor angetrieben wird. Dadurch ist keine gasdichte Durchführung des Seiles durch die Behälterwand erforderlich.

Der beschriebene Stand der Technik funktioniert sehr zufriedenstellend. Bei der Verwendung von Betondächern ist allerdings der konstruktive Aufwand für eine solche Biogasanlage relativ hoch. Ein Serviceschacht an einem flexiblen Foliendach ermöglicht eine wesentlich leichtere Wartung eines Rührgerätes und ermöglicht zudem auch eine erleichterte Verstellung des Rührgerätes. Der konstruktive Aufwand für einen solchen Wartungsschacht erhöht aber auch den Preis.

Sind auch günstigere Fermenter bekannt geworden, so offenbart beispielsweise die DE 2009 001 118 U1 eine Verstelleinrichtung für Tauchmotorrührwerke, bei der ein Seil zur Höhenverstellung des Tauchmotorrührwerkes durch ein als Kolbenstange ausgeführtes Rohr durch die Behälterwand nach außen durchgeführt wird. Außen and er Behälterwand ist eine Handseilwinde vorgesehen, durch deren Betätigung das Tauchmotorrührwerk abgesenkt oder hochgezogen werden kann. Durch eine axiale Verstellung der Kolbenstange wird weiterhin der Schwenkwinkel des Tauchmotorrührwerkes beeinflusst. Grundsätzlich funktioniert auch ein solcher Stand der Technik. Um die Seildurchführung durch die Behälterwand abzudichten, ist aber eine regelmäßige Schmierung der Kolbenstange als Führungsrohr erforderlich, um die Gasdichtheit zu gewährleisten. Durch die Längsbewegung des Seiles durch als Führungsrohr dienende Kolbenstange ist es schwierig, bei dieser Konstruktion für die geforderte Dichtheit zu sorgen. In anderen Konstruktionen ist es bekannt geworden, ein solches Führungsseil durch eine Wassertasse oder dergleichen zu leiten, um nach dem Prinzip eines Siphons die Gasdichtheit zu gewährleisten. Eine solche Wassertasse muss regelmäßig überprüft werden, da es einerseits insbesondere im Sommer zu einem Austrocknen der Wassertasse kommen kann, während es bei starken Minusgraden zu einem Einfrieren der Wassertasse kommen kann.

Ein weiterer Nachteil des Standes der Technik gemäß der DE 2009 001 118 U1 und vergleichbarer Fermenterkonstruktione4n ist, dass das Rührgerät für die Wartung zwar nach oben gezogen werden kann, aber auch in der obersten Stellung sich noch weiter unterhalb des oberen Endes der Behälterwandung befindet. Deshalb muss der Substratspiegel vor einer Wartung gegebenenfalls abgesenkt werden und die Durchführung der Wartung ist erheblich schwieriger als bei der Verwendung eines Serviceschachtes gemäß der EP 1 171 305 B1.

Es ist deshalb die Aufgabe der vorliegenden Erfindung einen Fermenterbehälter zur Verfügung zu stellen, der kostengünstig herstellbar ist und bei dem eine einfache Wartung der Rühreinheit ermöglicht wird.

Diese Aufgabe wird gelöst durch den Fermenter mit den Merkmalen des Anspruchs 1 und durch eine Rühreinrichtung mit den Merkmalen des Anspruchs 26. Bevorzugte Weiterbildungen sind Gegenstand der Unteransprüche. Weitere Vorteile ergeben sich aus der allgemeinen Beschreibung und der Beschreibung der Ausführungsbeispiele.

Ein erfindungsgemäßer Fermenter ist insbesondere für eine Biogasanlage vorgesehen und weist wenigstens eine Fermenterwandung und wenigstens einen Fermenterinnenraum auf. Der Fermenter weist wenigstens Fermenterdach auf, welches wenigstens teilweise zur Horizontalen geneigt ist und welches insbesondere flexibel ausgebildet ist. Es ist wenigstens eine Rühreinrichtung vorgesehen, um ein in dem Fermenterinnenraum vorhandenes Substrat umzurühren. Die Rühreinrichtung umfasst wenigstens eine Führungseinheit, wenigstens eine Stützeinheit und wenigstens eine an der Führungseinheit aufgenommene verstellbare Rühreinheit. Die Stützeinheit zur Abstützung der Führungseinheit ist an der Fermenterwandung befestigt. Die Rühreinheit ist höhenverstellbar vorgesehen. Es ist wenigstens eine Einstelleinrichtung zur Verstellung der Rühreinheit vorgesehen. Das obere Ende der Führungseinheit erstreckt sich bis oberhalb eines oberen Endes der Fermenterwandung und endet unterhalb des Fermenterdaches innerhalb des Fermenterinnenraumes.

Der erfindungsgemäße Fermenter hat viele Vorteile. Ein erheblicher Vorteil des erfindungsgemäßen Fermenters besteht darin, dass ein einfacher Betrieb und eine einfache Wartung ermöglicht werden. Die Führungseinheit kann z. B. eine Führungsstange bzw. einen Stützmast und auch eine Getriebeeinrichtung und/oder eine Abstützung oder Halterung umfassen, wobei sich die Führungseinheit bis oberhalb der oberen Kante der Fermenterwandung erstreckt. Da das Fermenterdach unter einem entsprechenden Winkel zum Zentrum hin ansteigt, kann die Führungseinheit über die Oberkante der Fermenterwandung hinaus ragen, ohne durch das Fermenterdach hindurch zu stoßen. Durch diese konstruktiven Maßnahmen wird es ermöglicht, dass die Rühreinheit weiter nach oben verstellt werden kann, als dies bei anderen Konstruktionen der Fall ist. Bei anderen Konstruktionen aus dem Stand der Technik, die über keinen Serviceschacht verfügen, enden die Führungseinheiten unterhalb der Oberkante der Fermenterwandung. Das führt dazu, dass der Fermenter wenigstens zum Wartungszeitpunkt nicht so weit gefüllt werden kann, wie es ansonsten möglich wäre. Das führt dazu, dass vor einer Wartung erst aufwändig Substrat aus dem Fermenter entfernt werden muss. Andernfalls wird die Kapazität des Fermenters nicht soweit ausgenutzt, wie es möglich wäre. Durch die Erfindung werden deshalb eine höhere Ausnutzung und ein geringerer Betriebsaufwand ermöglicht.

Erfindungsgemäß ist wenigstens ein Teil der Rühreinheit bis über das obere Ende der Fermenterwandung verstellbar. Insbesondere ist wenigstens ein Rührflügel wenigstens teilweise über das obere Ende der Fermenterwandung verstellbar. Besondere bevorzugt ist aber wenigstens ein Teil des Gehäuses der Rühreinheit bis über das obere Ende der Fermenterwandung verstellbar vorgesehen. Dadurch wird eine besonders einfache Wartung der Rühreinheit ermöglicht.

Besonders bevorzugt ist die Rühreinheit so hoch verstellbar, dass die Drehachse der Rühreinheit bis über das obere Ende der Fermenterwandung hinaus steht. In besonders bevorzugten Ausgestaltungen ist die Rühreinheit im Wesentlichen und insbesondere vollständig bis oberhalb des oberen Endes der Fermenterwandung verstellbar. Solche Ausgestaltungen ermöglichen eine besonders einfache Zugänglichkeit der Rühreinheit.

Vorzugsweise umfasst die Stützeinheit wenigstens eine sich von der Fermenterwandung aus schräg nach oben erstreckende Haltestange. Insbesondere sind wenigstens zwei Stützeinheiten vorgesehen, die sich jeweils von der Fermenterwandung aus schräg nach oben erstrecken. Dabei weist jede Stützeinheit insbesondere wenigstens eine Haltestange auf. In der Draufsicht gesehen ist es bevorzugt, dass die beiden Stützeinheiten sich von der Führungseinheit v-förmig aus auf die Behälterwandung zu erstrecken. Dadurch wird eine stabile Konstruktion ermöglicht, während gleichzeitig die Rühreinheit zwischen den beiden Stützeinheiten nach oben verfahren werden kann.

Erfindungsgemäß umfasst die Einstelleinrichtung wenigstens eine außerhalb des Fermenterinnenraums angeordnete Bedieneinheit und wenigstens eine innerhalb des Fermenterinnenraums angeordnete Einstelleinheit. Die Bedieneinheit ist dabei insbesondere mit der Einstelleinheit über eine gasdichte Durchführungseinrichtung gekoppelt. Die Durchführungseinrichtung kann als Getriebeeinheit ausgebildet sein. In einfachen Fällen handelt es sich bei der Durchführungseinrichtung um eine gasdicht aufgenommene drehbare Welle. Rotationsbewegungen sind einfacher gasdicht zu halten als eine Lineardurchführung eines Seiles.

Vorzugsweise umfasst die Führungseinheit wenigstens einen Stützmast und wenigstens eine Getriebeeinrichtung. Dabei kann die Getriebeeinrichtung insbesondere auch ein anderes oder separates Anbauteil sein, welches oben auf dem Stützmast befestigt bzw. angebracht ist.

Bevorzugt dient die Getriebeeinheit dazu, die Rührwerkeinrichtung entlang des Stützmastes in der Höhe zu verstellen und/oder das Rührwerk an dem Stützmast zu verschwenken. Dabei können auch andere Mittel eingesetzt werden, die dazu geeignet sind, eine Verstellung bzw. ein Verschwenken durchzuführen, die kein Getriebe im eigentlichen Sinne darstellen.

In bevorzugten Ausgestaltungen steht die Einstelleinheit mit der Getriebeeinrichtung in Wirkverbindung. So kann die Rührwerkeinheit über die Bedieneinheit außerhalb des Fermenters einfach verstellt werden.

Bevorzugt ist die Einstelleinheit mechanisch, hydraulisch und/oder pneumatisch ausgebildet. Dann kann die Rührwerkeinheit über die Bedieneinheit z. B. mittels Stangen bzw. einem Stangengetriebe oder auch über hydraulischen oder pneumatischen Druck verstellt oder verschwenkt werden.

Besonders bevorzugt ist die Getriebeeinrichtung als Hydraulikgetriebe ausgebildet und die Einstelleinheit ist als wenigstens ein Hydraulikschlauch vorgesehen. Der Hydraulikschlauch verbindet dann vorzugsweise das Hydraulikgetriebe mit der Bedieneinheit, wobei die Bedieneinheit insbesondere wenigstens eine Hydraulikpumpe umfasst. Diese Hydraulikpumpe kann z. B. fest installiert sein und als Handpumpe oder als motorisierte Pumpe ausgebildet sein. An der Bedieneinheit kann bevorzugt aber auch eine Schnittstelle vorgesehen sein, an die eine Hydraulikpumpe angeschlossen werden kann. An diese Schnittstelle können dann beispielsweise auch Hydraulikschläuche angeschlossen werden, die an eine beliebige Stelle geführt werden können.

Dann kann z. B. auf dem Boden neben dem Fermenter eine mobile Hydraulikpumpe vorgesehen sein. Dies eignet sich insbesondere auch zu Wartungszwecken. Bevorzugt ist auch, dass die an die Schnittstelle angeschlossenen Hydraulikschläuche in einen Technikraum geleitet werden, wo sie an eine motorisierte Hydraulikpumpe angeschlossen werden.

In anderen bevorzugten Ausgestaltungen ist die Einstelleinrichtung mechanisch ausgebildet, wobei die Einstelleinheiten insbesondere als Stangen ausgeführt sind. Dann umfasst die die Bedieneinheit vorzugsweise eine Kurbeleinrichtung, welche als Handkurbel oder auch als Schnittstelle zum Anschluss einer motorisierten Kurbeleinrichtung ausgebildet sein kann.

In allen Ausgestaltungen ist es besonders bevorzugt, dass die gasdichte Durchführungseinrichtung unterhalb des Fermenterdaches und insbesondere in der Fermenterwandung angeordnet ist. Die gasdichte Durchführungseinrichtung erlaubt einen einfachen und zuverlässigen Betrieb des Fermenters. Das Foliendach kann unabhängig von der vorgesehenen Wartungsfunktion konstruiert werden. Es ist nicht nötig, eine gasdichte Durchführung von Komponenten durch das Foliendach vorzusehen. Eine gasdichte Durchführungseinrichtung kann auf einfache Art und Weise in die Behälterwandung bzw. in die Fermenterwandung integriert werden.

Erfindungsgemäß liegt die Bedieneinheit unterhalb des oberen Endes der Führungseinheit. Insbesondere ist die Bedieneinheit auch unterhalb des oberen Endes der Fermenterwandung angeordnet. Bei einer solchen Ausgestaltung ist die Bedieneinheit unterhalb des oberen Endes der Führungseinheit vorgesehen.

Insbesondere zur Überbrückung einer solchen hohen Differenz kann die Einstelleinheit wenigstens eine Umlenkeinrichtung umfassen. Die Umlenkeinrichtung kann wenigstens eine Kardangelenkeinrichtung und/oder wenigstens eine Winkelantriebseinrichtung und/oder wenigstens eine biegsame Welle umfassen oder als eine solche ausgebildet sein. Eine solche Umlenkeinrichtung in Form z. B. einer Kardangelenkeinrichtung oder dergleichen ermöglicht eine zuverlässige und einfache Übertragung von Drehbewegungen, die unter einem Winkel zueinander stehen. Dadurch kann von der gasdichten Durchführungseinrichtung über die Umlenkeinrichtung eine sich nach oben erstreckende Koppeleinheit angekoppelt werden, die zur Horizontalen geneigt angeordnet ist. Dadurch können manuelle Bedienungen an der Bedieneinheit aus dem Bereich der Behälterwandung nach oben hin übertragen werden.

Die Koppeleinheit ist insbesondere zwischen der Umlenkeinrichtung in Form von z. B. einer Kardangelenkeinrichtung und der Durchführungseinrichtung angeordnet.

In allen Ausgestaltungen ist die Rühreinheit insbesondere verschwenkbar vorgesehen. Dabei ist der Schwenkwinkel über eine Winkeleinstelleinrichtung einstellbar. Die Einstelleinrichtung für den Winkel kann in analoger Weise ausgestaltet sein, sodass neben der Einstelleinrichtung für die Höhe eine zweite Einstelleinrichtung für den Winkel vorgesehen ist.

In allen Ausgestaltungen ist vorzugsweise ein Abstand der Führungseinheit von der Innenseite der Fermenterwandung so bemessen, dass die Rühreinheit zur Fermenterwandung hin verschwenkbar ist. Dabei ist der Abstand der Führungseinheit von der Innenseite der Fermenterwandung größer als die entsprechende Erstreckung der Rühreinheit. Besonders bevorzugt ist die Rühreinheit um etwa 360° verschwenkbar ausgeführt. Dabei kann der Schwenkwinkel kleiner oder größer als 360° betragen.

Vorzugsweise ist an der Außenseite der Fermenterwandung wenigstens eine Plattform vorgesehen und insbesondere dort an der Außenseite befestigt. Eine solche Plattform kann beispielsweise für die Wartung der Rühreinheit genutzt werden. Dann kann sich eine Bedienperson auf die Plattform stellen und von dort die Rühreinheit warten. Es ist bevorzugt, dass wenigstens eine Plattform an wenigstens einer Stützeinheit insbesondere entfernbar angeordnet ist.

Erfindungsgemäß ist in dem Fermenterdach wenigstens eine gasdicht verschließbare Serviceöffnung vorgesehen. Insbesondere ist eine solche verschließbare Serviceöffnung oberhalb der Führungseinheit vorgesehen. Eine solche Ausgestaltung ermöglicht eine besonders einfache und schnelle Wartung des Rührgerätes, da das Rührgerät bzw. die Rühreinheit nach oben verfahren wird und anschließend die verschließbare Serviceöffnung geöffnet wird, sodass die Rühreinheit von außerhalb zugänglich ist.

Die Serviceöffnung umfasst bevorzugt einen Servicerahmen oder wird in einem Servicerahmen gebildet. So kann auf einfache Art und Weise eine Serviceöffnung in ein Foliendach eingebracht werden.

In vorteilhaften Ausgestaltungen ist wenigstens eine Schutzbügeleinrichtung unterhalb des Fermenterdaches vorgesehen ist. Dabei ist die Schutzbügeleinrichtung besonders bevorzugt wenigstens abschnittsweise oberhalb der Führungseinrichtung angeordnet.

Die Schutzbügeleinrichtung kann insbesondere als Schutzbügel ausgestaltet sein, der bevorzugt mit seinen beiden Enden an der Fermenterwand befestigt ist und auch mit der Führungseinheit verbunden sein kann. Die Schutzbügeleinrichtung kann aber in anderen vorteilhaften Ausgestaltungen nicht mit der Fermenterwandung verbunden sein, sondern nur an der Führungseinrichtung aufgenommen sein.

Dabei deckt die Schutzbügeleinrichtung insbesondere den oberen Teil der Führungseinheit ab, sodass das Foliendach vor einer eventuellen Beschädigung durch die Führungseinrichtung geschützt ist, falls das Dach durch Druckverlust einfällt.

Der Schutzbügel kann aber in anderen bevorzugten Ausgestaltungen auch auf dem oberen Bereich der Führungseinheit vorgesehen sein und insbesondere ausschließlich an dieser befestigt sein. Auch so kann ein ausreichender Schutz des Foliendaches erreicht werden.

Bevorzugt ist der Servicerahmen an der Schutzbügeleinrichtung aufgenommen. So kann der Servicerahmen Spannungen im Foliendach abfangen und es wird eine besonders stabile Serviceöffnung bereitgestellt.

In allen zuvor beschriebenen Ausgestaltungen ist es bevorzugt, dass die Einstelleinrichtung händisch und/oder motorisiert betreibbar ist. So können je nach Budget auch günstigere Anlagen zusammengestellt werden und es ist zudem möglich, die Rührwerkeinrichtung auch im Falle eines Stromausfalls oder Motordefekts zu verstellen.

Die erfindungsgemäße Rühreinrichtung ist insbesondere für den Einsatz an einem Fermenter für eine Biogasanlage vorgesehen, um ein in dem Fermenterinnenraum vorhandenes Substrat umzurühren. Die Rühreinrichtung umfasst wenigstens eine Führungseinheit und wenigstens eine Stützeinheit. An der Führungseinheit ist eine verstellbare Rühreinheit aufgenommen und die Stützeinheit ist zur Abstützung der Führungseinheit an einer Fermenterwandung befestigt bzw. befestigbar. Es ist wenigstens eine Einstelleinrichtung zur Verstellung der Rühreinheit vorgesehen. Die Einstelleinrichtung umfasst wenigstens eine Einstelleinheit, die über wenigstens eine gasdichte Durchführungseinrichtung mit wenigstens einer Bedieneinheit verbunden ist.

Die erfindungsgemäße Rühreinrichtung hat ebenfalls viele Vorteile, da sie einen einfachen und zuverlässigen Betrieb mit geringem Aufwand ermöglicht.

Vorzugsweise ist eine Einstelleinheit zur Einstellung einer Höhe der Rühreinheit vorgesehen. Insbesondere ist eine Einstelleinheit zur Einstellung eines Winkels der Rühreinheit vorgesehen. Besonders bevorzugt ist die Rühreinheit höhenverstellbar und verschwenkbar angeordnet.

In zweckmäßigen Weiterbildungen ist wenigstens eine Einstelleinheit mechanisch, hydraulisch oder pneumatisch ausgebildet.

Die Durchführungseinrichtung kann insbesondere als Gelenkdurchführung ausgebildet sein oder eine solche umfassen.

In allen Ausgestaltungen umfasst wenigsten eine Einstelleinrichtung vorzugsweise wenigstens eine Umlenkeinrichtung und/oder eine Kardangelenkeinrichtung.

Weitere Vorteile und Anwendungsmöglichkeiten der vorliegenden Erfindung ergeben sich aus dem Ausführungsbeispiel, welches im Folgenden mit Bezug auf die beiliegenden Figuren erläutert wird.

In den Figuren zeigen:
- Fig. 1: eine schematische Seitenansicht eines erfindungsgemäßen Fermenter;
- Fig. 2: einen schematischen Querschnitt durch das obere Ende der Fermenterwand und eine relativ weit nach oben gezogene Rühreinheit;
- Fig. 3: einen Querschnitt durch die gesamte Höhe der Fermenterwandung;
- Fig. 4: eine perspektivische Innenansicht eines weiteren Ausführungsbeispiels;
- Fig. 5: eine Außenansicht auf das Segment der Fermenterwandung aus Fig. 4;
- Fig. 6: eine Frontalansicht einer weiteren Ausführung eines erfindungsgemäßen Fermenters in einer rein schematischen Darstellung;
- Fig. 7: eine perspektivische Ansicht des Fermenters gemäß Fig. 6 in einer rein schematischen Darstellung;
- Fig. 7a: eine Detailvergrößerung des gestrichelt markierten Bereichs in Fig. 7;
- Fig. 8: eine perspektive Innenansicht des erfindungsgemäßen Fermenters gemäß Fig. 6 und 7 in einer rein schematischen Darstellung;
- Fig. 9: eine Frontansicht auf eine andere Ausführung eines erfindungsgemäßen Fermenters;
- Fig. 10: einen Teilbereich des Fermenters gemäß Fig. 9 in einer schematischen Seitenansicht;
- Fig. 11: eine perspektivische Ansicht auf eine Durchführungseinrichtung in einer rein schematischen Darstellung;
- Fig. 12: die Durchführungseinrichtung gemäß Fig. 11 in einer rein schematischen Rückansicht; und
- Fig. 13: die Durchführeinrichtung gemäß Fig. 11 und 12 in einer rein schematischen Seitenansicht.

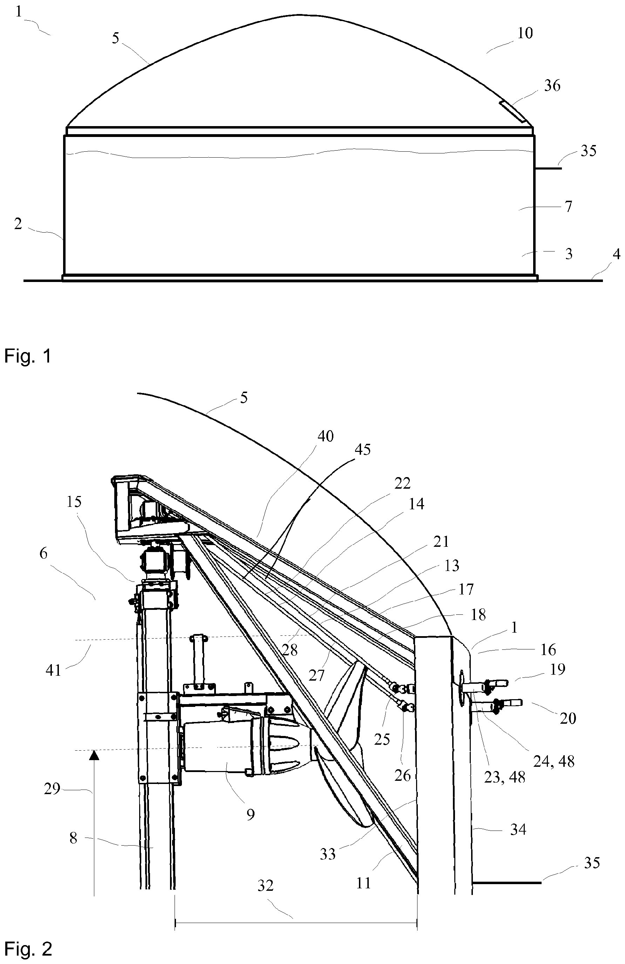

Mit Bezug auf die Figuren werden nun zwei Ausführungsbeispiele erläutert. Dabei zeigt Figur 1 in einer stark schematischen Seitenansicht einen Fermenter 1 einer Biogasanlage 10. Der Fermenter 1 weist vorzugsweise einen etwa kreisförmigen Querschnitt auf und verfügt über eine hier umlaufende Fermenterwandung 2. Das Fermenterdach 5 wird durch ein insbesondere flexibles Material gebildet und erstreckt von dem Rand aus nach oben, sodass sich eine gewölbte und z. B. viertel- oder halbkugelförmige oder sonst wie gebogene Struktur des Behälterdaches 5 ergibt. Der Neigungswinkel des Fermenterdaches 5 hängt von den konkreten Bedingungen ab und kann 15° oder mehr und insbesondere auch 30 oder 45° erreichen oder überschreiten. Vorzugsweise ist das Fermenterdach 5 wenigstens teilweise und insbesondere vollständig entfernbar, um den Fermenterinnenraum 3 zugänglich zu machen.

In dem Fermenterinnenraum 3 ist im Betrieb ein Substrat 7 vorgesehen.

In dem Fermenterdach 5 kann wenigstens eine Serviceöffnung 36 vorgesehen sein, um beispielsweise ein in dem Fermenterinnenraum 3 angeordnetes Rührgerät zu warten. Eine Plattform 35 kann beispielsweise an der Außenseite 34 oder der Innenseite 33 der Fermenterwandung 2 befestigt sein, damit eine Bedienperson sich darauf stellen kann.

Figur 2 zeigt einen Teilquerschnitt durch die Fermenterwandung 2, wobei die Rühreinrichtung 6 mit der daran vorgesehenen Rühreinheit 9 sichtbar ist.

Die Rühreinheit 9 ist als Tauchmotorrührgerät ausgeführt und kann elektrisch oder beispielsweise hydraulisch angetrieben werden. Die Rühreinheit 9 verfügt hier im Ausführungsbeispiel über eine Mehrzahl von z. B. drei Rührflügeln 38, die sich im Betrieb der Rühreinheit 9 drehen und somit für eine Durchmischung des Substrates 7 in dem Fermenterinnenraum 3 führen. Dadurch werden eine Homogenisierung und eine bessere Effektivität ermöglicht.

Die Rühreinheit 9 ist an einer Führungseinheit 8 höhenverstellbar gehalten, wobei die Führungseinheit 8 in dem hier gezeigten Ausführungsbeispiel einen Stützmast 8a und eine Getriebeeinrichtung 8b umfasst. Der Stützmast 8a wird hier durch ein Hohlprofil zur Verfügung gestellt.

Die Rühreinheit 9 kann aus der in Figur 2 dargestellten Position soweit nach oben gefahren werden, bis die Rühreinheit 9 an dem oberen Ende 15 der Führungseinheit 8 anschlägt. Die Rühreinheit 9 kann nach unten ab entlang der Führungseinheit 8 abgesenkt werden, bis die Rührflügel 38 nur noch einen kurzen Abstand oberhalb des Fermenterbodens erreichen.

Die Rühreinheit 9 ist hier zusammen mit dem etwa rechteckig ausgebildeten Stützmast bzw. der etwa rechteckig oder mit einem sonstigen Querschnitt ausgebildeten Führungseinheit verschwenkbar vorgesehen. Zur Verschwenkung der Rühreinheit 9 und zur Einstellung eines Rührwinkels kann die Führungseinheit 8 um ihre Längsachse gedreht werden. Dadurch wird es ermöglicht, dass die Rühreinheit 9 in jede beliebige Richtung ausgerichtet ist.

Die Rühreinrichtung 6 umfasst neben der Führungseinheit 8 auch noch Stützeinheiten 11 und 12, die insgesamt ein Stützgestell bilden, mit dem die Rühreinrichtung 6 an der Innenseite 33 der Fermenterwandung 2 befestigt und gehalten ist. Dabei umfassen die Stützeinheiten 11 und 12 jeweils eine oder mehrere Haltestangen 17 und 18, die hier jeweils schräg nach oben verlaufen. Dadurch wird eine effektive Abstützung der Führungseinheit 8 ermöglicht, während gleichzeitig die Rühreinheit 9 weiter nach oben verfahren werden kann, als dies bei den bislang bekannten Rühreinrichtungen der Fall ist.

Die Führungseinheit 8 bzw. deren oberes Ende 15 ist in dem Fermenterinnenraum 3 angeordnet. Das bedeutet, dass die Führungseinheit 8 des Fermenterdaches 5 nicht durchstößt. Die Führungseinrichtung 6 bleibt insgesamt unterhalb des Fermenterdaches 5. Das ermöglicht einen erheblich einfacheren Aufbau des Fermenterdaches 5, da dessen Konstruktion nicht für die zusätzlichen Belastungen ausgelegt werden muss. Es wird der Einsatz eines Standarddaches ermöglicht, was den Kostenaufwand erheblich senkt. Gleichzeitig wird es durch die erfindungsgemäße Konstruktion ermöglicht, die Rühreinheit 9 über das obere Ende 16 der Fermenterwandung 2 hinauszufahren, um es in die Wartungsposition zu bringen.

Der Abstand 32 der Führungseinheit 8 von der Innenseite 33 der Fermenterwandung 2 ist so bemessen, dass die Rühreinheit 9 auf die Fermenterwandung 2 zugeschwenkt werden kann. Hier ist es möglich, die Rühreinheit 9 vollständig zu verschwenken. Dadurch sind die Rührflügel 38 in der Wartungsstellung möglichst nahe an der Fermenterwandung 2 angeordnet, sodass ein einfacher Service ermöglicht wird.

Dadurch, dass in der Wartungsstellung die Rührflügel 38 wenigstens teilweise über das obere Ende 16 der Fermenterwandung 2 hinausragen, kann ein auf der Plattform 35 stehender Servicetechniker die Rührflügel 38 relativ bequem tauschen.

Das Stützgestell, bestehend aus den Stützeinheiten 11 und 12 mit den Haltestangen 17 und 18 ist nur an der Fermenterwandung 2 befestigt. Eine Verbindung zu dem Fermenterdach 5 besteht hier im Ausführungsbeispiel nicht.

Um eine Höhe 29 der Rühreinheit 9 einzustellen, und um einen Schwenkwinkel 30 der Rühreinheit 9 einzustellen, sind Einstelleinrichtungen 13 und 14 vorgesehen. Dabei dient die Einstelleinrichtung 13 zur Einstellung der Höhe 29 der Rühreinheit 9 und die Einstelleinrichtung 14 dient zur Einstellung des Schwenkwinkels 30 der Rühreinheit 9. Grundsätzlich ist es auch möglich, dass die Einstelleinrichtung 13 zur Einstellung des Schwenkwinkels und die Einstelleinrichtung 14 zur Einstellung einer Höhe 29 der Rühreinheit 9 vorgesehen sind.

Die Höhe 29 der Drehachse 39 der Rühreinheit 9 kann bis zu der maximalen Höhe 41 eingestellt werden, wenn das Gehäuse der Rühreinheit 9 an das obere Ende anschlägt. Die maximale Höhe 41 der Drehachse 39 ist hier höher als das obere Ende 16 der Fermenterwandung 2. Gegebenenfalls kann die Rühreinheit 9 insgesamt höher verfahren werden als das obere Ende 16 der Fermenterwandung 2.

Beide Einstelleinrichtungen 13 und 14 sind hier im Wesentlichen vergleichbar aufgebaut.

Die Einstelleinrichtung 13 umfasst eine Bedieneinheit 19 an der Außenseite 34 der Fermenterwandung 2. An die Bedieneinheit 19 schließlich sich eine gasdichte Durchführungseinrichtung 23 an, die durch die Fermenterwandung 2 gasdicht hindurchführt. Die Durchführungseinrichtung ist bei dem hier gezeigten Fermenter als Gelenkdurchführung 48 ausgebildet. Dabei schließt sich auf der Innenseite 33 der Fermenterwandung an die gasdichte Durchführungseinrichtung 23 eine Kardangelenkeinrichtung als Umlenkeinrichtung 25 an, die für eine Winkelumlenkung der Drehbewegung sorgt. Von der Kardangelenkeinrichtung als Umlenkeinrichtung 25 aus erstreckt sich die Koppeleinheit 27 aus schräg nach oben. Die Kardangelenkeinrichtung als Teil der Umlenkeinrichtung 25 bildet mit der Koppeleinheit 27 eine Einstelleinheit 21, welche hier mechanisch ausgebildet ist und eine Stange 45 umfasst.

Die Einstelleinrichtung 14 umfasst eine außen an der Fermenterwandung 2 angeordnete Bedieneinheit 20, die über eine gasdichte Durchführungseinrichtung durch die Fermenterwandung 2 hindurchführt.

Auf der Innenseite 33 der Fermenterwandung 2 ist eine Kardangelenkeinrichtung als Umlenkeinrichtung 26 vorgesehen, die Teil einer Einstelleinheit 22 ist. Auch die Einstelleinheit 22 ist hier mechanisch ausgebildet ist und umfasst eine Stange 45. Die Kardangelenkeinrichtung als Teil der Umlenkeinrichtung 26 ist mit einer schräg nach oben führenden Koppeleinheit 28 verbunden.

Die Koppeleinheiten 27 und 28 sind an ihren oberen Enden mit Getriebeelementen oder Getriebeeinrichtungen verbunden, die eine Umlenkung der Koppelbewegungen bewirken. Dadurch wird die Drehbewegung der Bedieneinheit 19 in eine Höhenverstellung der Rühreinheit 9 und eine Drehbewegung der Bedieneinheit 20 in einer Schwenkbewegung der Führungseinheit 8 und somit auch der Rühreinheit 9 umgesetzt.

Die Rühreinheit 9 kann über ein Seil an dem oberen Ende 15 der Führungseinheit 8 gehalten werden, wo das Seil auf eine Rolle aufgewickelt wird. Die Drehung der Rolle wird über eine Drehung der Bedieneinheit 19 bewirkt.

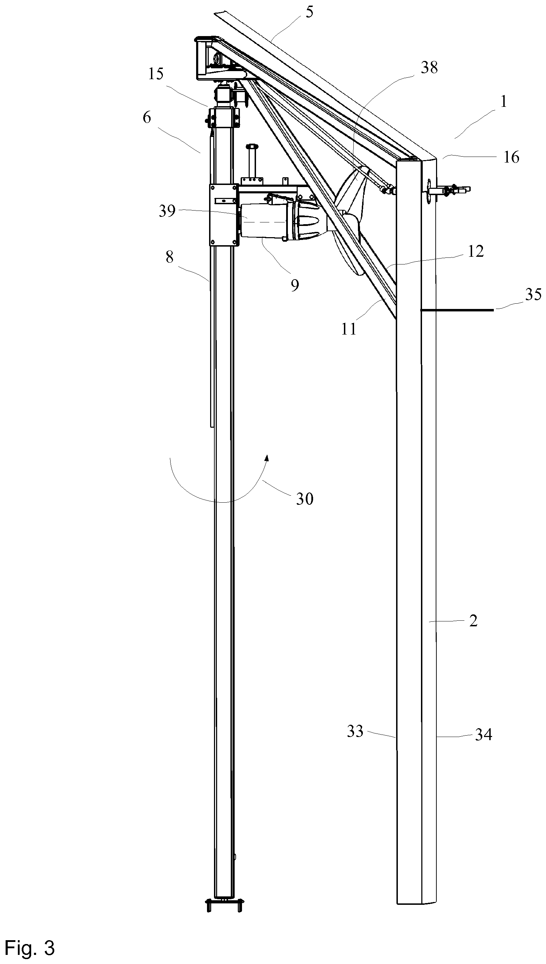

Figur 3 zeigt die vollständige Höhe der Fermenterwandung 2. Die Führungseinheit 8 ist am unteren Ende drehbar an dem Fermenterboden aufgenommen, um eine Verschwenkung der Rühreinheiten 9 zu ermöglichen. Erkennbar ist auch, dass das Fermenterdach 5 oberhalb der Rühreinrichtung 6 verläuft und nicht mit dieser verbunden ist.

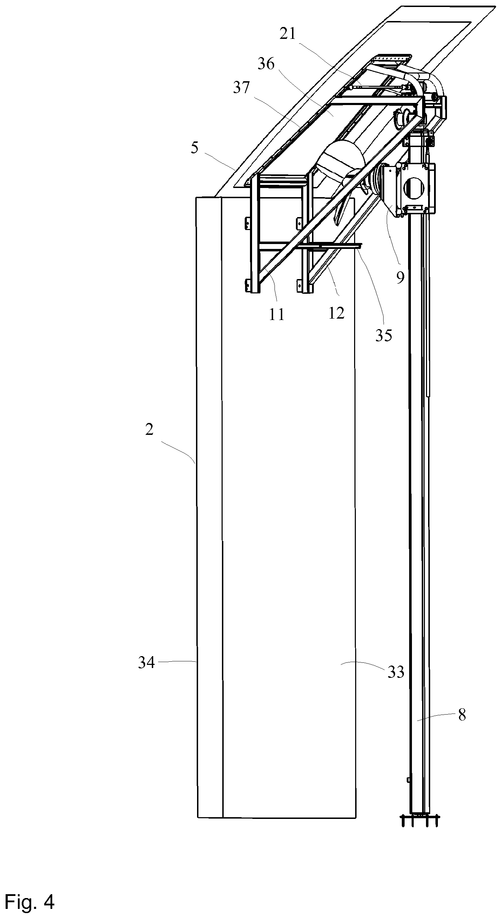

Figur 4 zeigt ein anderes Ausführungsbeispiel, wobei eine perspektivische Innenansicht eines Abschnitts der Fermenterwandung 2 zu sehen ist. Auch hier ist die Rühreinheit 9 höhenverstellbar an einer Führungseinheit 8 angeordnet, die schwenkbar auf dem Fermenterboden angebracht ist. Am oberen Ende wird die Führungseinheit 8 über ein Stützgestell gehalten. Das Stützgestell 40 umfasst hier zwei Stützeinheiten 11 und 12, die jeweils eine oder mehrere Haltestangen 17 und 18 aufweisen. Möglich ist es aber auch, dass nur eine Stützeinheit und kein Stützgestell vorgesehen ist. Dazu kann die Stützeinheit beispielsweise scheibenartig ausgebildet sein und die Führungseinheit 8 schwenkbar führen.

Hier kann auch im Inneren des Fermenters 1 eine Plattform 35 an den Stützeinheiten 11 und 12 vorgesehen sein oder bei Bedarf positioniert werden. Die Plattform kann gegebenenfalls dazu dienen, dass sich eine Bedienperson darauf stellt. Es ist aber auch möglich, dass die Plattform 35 dazu dient, Gegenstände während der Wartungsarbeiten dort abzulegen.

Im Unterschied zum Ausführungsbeispiel gemäß der Figuren 1 und 2 umfasst die Rühreinrichtung 6 hier einen Servicerahmen 37, der in das Fermenterdach 5 integriert ist und der die Serviceöffnung 36 zur Verfügung stellt. Der Servicerahmen 37 wird von den Stützeinheiten 11 und 12 getragen.

In dieser Ausgestaltung ist es möglich, dass die Einstelleinheiten 21 und 22 zur Höheneinstellung und zur Winkeleinstellung der Rühreinheit 9 nicht nach unten zur Fermenterwandung 2 führen, sondern direkt auf den Servicerahmen 37 ausgerichtet sind, wo entsprechende gasdichte Durchführungseinrichtungen 23 und 24 vorgesehen sind, mit der die Drehbewegung zur Einstellung der Rühreinheit 9 von außen nach innen übertragen wird.

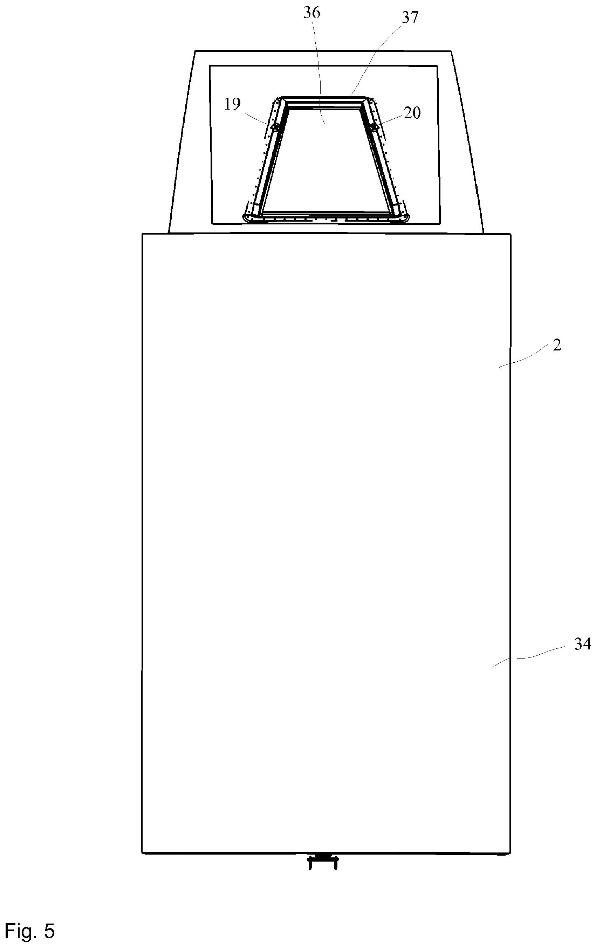

Figur 5 zeigt eine Außenansicht auf das Segment der Fermenterwandung 2 gemäß Figur 4, wobei oberhalb des oberen Endes 16 der Fermenterwandung 2 der Servicerahmen 37 mit der Serviceöffnung 36 erkennbar ist. Des Weiteren sind an dem Servicerahmen 37 die Bedieneinheiten 19 und 20 zu erkennen. Gegebenenfalls kann auch auf die hier sichtbaren Komponenten eine mechanische Drehkurbel aufgesteckt werden, um eine Höhen- und Schwenkverstellung der Rühreinheit 9 zu bewerkstelligen.

In allen Ausgestaltungen kann an den Bedieneinheiten 19 und 20 wenigstens eine Anzeige vorgesehen sein, aus der der Schwenkwinkel 30 bzw. die Höhe 29 der Rühreinheiten 9 erkennbar ist.

Insgesamt stellt die Erfindung einen Fermenter 1 bzw. eine Rühreinrichtung 6 zur Verfügung, womit ein kostengünstiger Aufbau und ein einfacher Betrieb ermöglicht werden. Mit der Erfindung ist es nicht nötig, die Serviceöffnung 36 in das Behälterdach 5 zu integrieren. Es ist auch möglich, dass die Rühreinrichtung 6 sich vollständig unterhalb des Fermenterdaches 5 erstreckt. Trotzdem kann die maximale Höhe der Rühreinheit 9 so eingestellt werden, dass die Rühreinheit 9 bis über das obere Ende 16 der Fermenterwandung 2 hinausragt. Das vereinfacht die Wartung der Rühreinheit 9, insbesondere wenn oberhalb der Rühreinrichtung 6 eine Serviceöffnung in dem Fermenterdach 5 zur Verfügung gestellt wird. In besonderen Ausgestaltungen ist es möglich, dass die Serviceöffnung 36 in einem Servicerahmen 37 zur Verfügung gestellt wird, die mit der Rühreinrichtung 6 verbunden ist.

Ein Vorteil der erfindungsgemäßen Konstruktion ist es auch, dass es keine Seildurchführungen durch die Behälterwand gibt. Das Rührgerät kann zur Wartung senkrecht an die Behälterwand geschwenkt werden. Zur Wartung muss der Füllstand nicht abgesenkt werden. Die Behälterwandung 2 kann im Betrieb praktisch bis zum Maximum gefüllt werden. Es ist möglich, dass eine Stellungsanzeige für die Rühreinheit 9 vorgesehen wird. Die Behälterausnutzung kann durch den maximal möglichen Füllstand erhöht werden, wodurch sich praktisch eine Baukostenreduktion ergibt.

Der Schwenkbereich der Rühreinheiten 9 kann 180°, 270°, 300°, 330° oder 360° betragen oder überschreiten. Zur Wartung kann die Rühreinheit 9 senkrecht an die Behälterwand geschwenkt werden. Gegebenenfalls kann die Rühreinrichtung 6 in das Fermenterdach 5 integriert werden.

Die erfindungsgemäße Konstruktion ist außerdem wartungsärmer, da die gasdichten Durchführungseinrichtungen dauerhaft dicht sind und nicht regelmäßig nachgeschmiert werden müssen. Auch ein Austrocknen einer Wassertasse ist ausgeschlossen.

Praktisch alle mechanischen Bauteile sind in dem Fermenterbehälter angeordnet. Gegebenenfalls kann auch wenigstens ein Sichtauge vorgesehen werden.

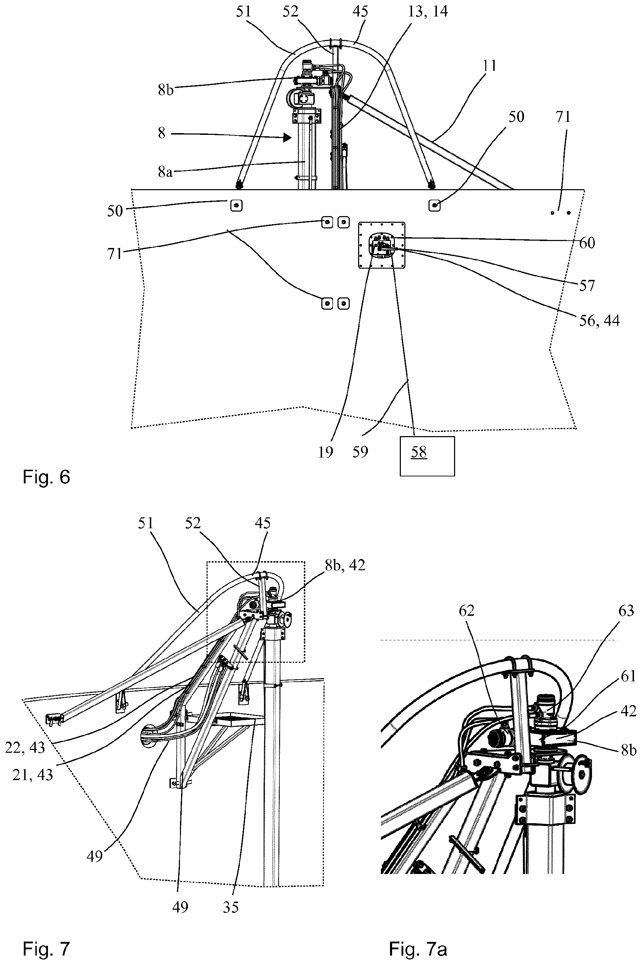

In den Figuren 6 bis 8 ist ein anderes Ausführungsbeispiel eines erfindungsgemäßen Fermenters 1 rein schematisch dargestellt. Dabei ähnelt der hier gezeigte Fermenter 1 im Grundaufbau dem zuvor beschrieben Fermenter 1.

In dem Fermenter 1 ist eine Führungseinheit 8 angeordnet, die hier einen Stützmast 8a und eine Getriebeeinrichtung 8b umfasst, wobei die Getriebeeinrichtung 8b als Hydraulikgetriebe 42 ausgebildet ist. Die Führungseinheit 8 wird über Konsole 49 an der Fermenterwandung 2 aufgenommen und in Position gehalten. Dabei werden die Konsole 49 und die Führungseinrichtung 8 mittels einer Stützeinheit 11 als seitliche Anstrebung abgestützt. An der Konsole 49 ist zudem eine Plattform 35 angebracht, auf der ein Techniker während der Wartung stehen kann.

Wie in den Figuren 6 und 7 schön zu sehen ist, ist oberhalb der Führungseinrichtung 8 eine Stützbügeleinrichtung 45 vorgesehen, die an zwei Befestigungsankern 50 mit der Fermenterwandung 2 fest verbunden ist. Die als Schutzbügel 51 ausgebildete Schutzbügeleinrichtung ist an der Führungseinheit 8 mittels einer Stütze 52 verbunden und abgestützt.

Dabei soll die Schutzbügeleinrichtung 45 eine Beschädigung des Fermenterdachs 5 vermeiden, wenn das Foliendach 5 beim Herunterfahren der Anlage oder im Falle einer Störung zusammenfällt.

Das Foliendach 5 fällt dann auf den Schutzbügel 51 und kann nicht durch die Führungseinheit 8 oder Teile davon beschädigt oder sogar durchlöchert werden.

In der Fermenterwand sind noch weitere Befestigungsanker zu erkennen, an denen die Konsole 49 und die Stützeinheit 11 an der Fermenterwandung 2 befestigt ist.

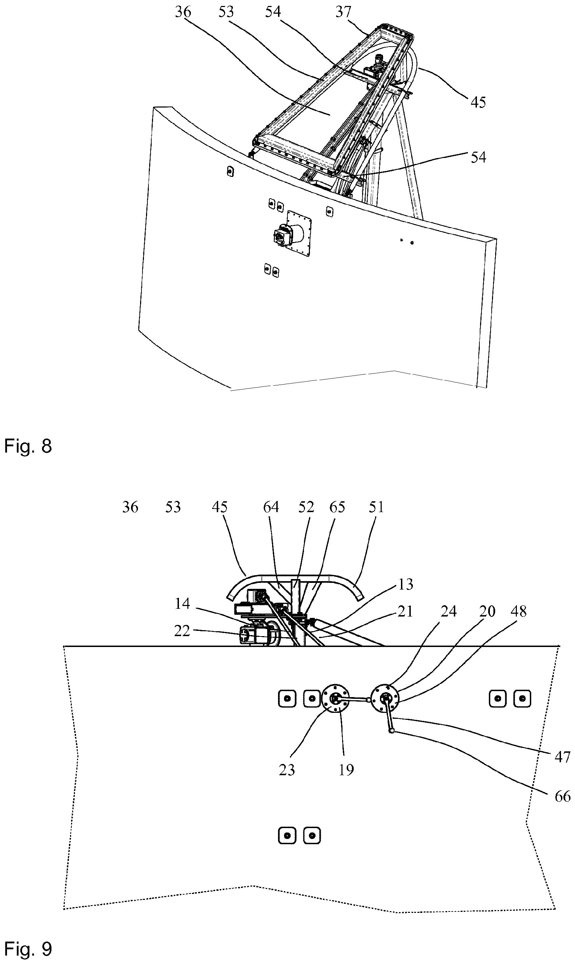

In Figur 8 ist weiterhin zu sehen, dass an der Schutzbügeleinrichtung 45 ein Servicerahmen 37 aufgenommen ist, um eine Serviceöffnung 36 bereitzustellen. Der Servicerahmen 37 ist in dem hier gezeigten Ausführungsbeispiel als Klemmrahmen 53 ausgebildet, der aus zwei Rahmenteilen besteht, zwischen welchen die verschiedenen Schichten des Foliendachs 5 eingeklemmt werden. So kann unter anderem auch nachträglich mit relativ einfachen Mitteln noch eine Serviceöffnung 36 in ein schon bestehendes Foliendach 5 eingebracht werden.

Um den Klemmrahmen 53 stabil aufzunehmen, sind in dem hier gezeigten Ausführungsbeispiel zwei Querträger 54 vorgesehen, die mit der Schutzbügeleinrichtung 45 verbunden sind und auf denen der Klemmrahmen 53 sicher aufliegt. Der Klemmrahmen 53 kann dann mit einem oder beiden Querträgern z. B. verschraubt werden. In anderen Ausgestaltungen kann der Klemmrahmen 53 auch anders an dem Fermenter 1 vorgesehen oder angebracht sein.

In den Figuren 6 bis 8 wird die Rühreinheit 9 hydraulisch verfahren und verschwenkt. Dazu ist die Getriebeeinrichtung 8b als Hydraulikgetriebe 42 ausgebildet, das mit hydraulischen Einstelleinrichtungen 13, 14 bedient wird.

Dabei ist das Hydraulikgetriebe 42 hier ein Doppelaufsatzgetriebe 61 mit zwei Getriebemodulen 62, 63 von denen das eine für die Höhenverstellung und das andere für die Verschwenkung der Rühreinheit 9 zuständig ist. Die Getriebemodule 62, 63 sind in der vergrößerten Darstellung in Figur 7a zu erkennen.

Die Einstelleinrichtungen 13, 14 umfassen als Hydraulikschläuche 43 ausgebildete Einstelleinheiten 21, 22, eine Energieversorgung 55 für die Rühreinheit 9 und eine gemeinsame Bedieneinheit 19.

Die Bedieneinheit 19 umfasst eine Hydraulikpumpe 44, mit welcher die Rühreinheit 9 hoch- und runtergefahren und verschwenkt werden kann.

Dazu ist an der Bedieneinheit 19 eine Handpumpe 56 mit einem Hebel 57 vorgesehen. Der Hebel 57 dient zum manuellen Betreiben der Einstelleinrichtungen 13, 14. Dazu kann mit dem Hebel 57 gepumpt werden, wobei der Hebel 57 in zwei Stellungen umgelegt werden kann, um die Rühreinheit 9 in der Höhe zu verstellen oder die Rühreinheit 9 zu verschwenken.

In allen Ausgestaltungen kann die Höhe und/oder der Schwenkwinkel der Rühreinheit(en) 9 auf einer hier nicht sichtbaren Positionsanzeige dargestellt werden.

Da die Bedieneinheit 19 in dem gezeigten Ausführungsbeispiel hoch oben am Fermenter 1 im explosionsgefährdeten Bereich angeordnet ist, ist der Einsatz von motorisierten Pumpen schwierig.

Daher kann an die Bedieneinheit 19 über eine Schnittstelle 60 eine motorisierte Pumpe 58 angeschlossen werden, die mit einer hydraulischen Leitung 59 aus dem Gefahrenbereich z. B. auf den Boden oder in einen Technikraum verlagert wurde. Auf dem Boden neben dem Fermenter 1 kann dann z. B. ein mobiler Motor bzw. eine mobile Hydraulikpumpe angeschlossen werden.

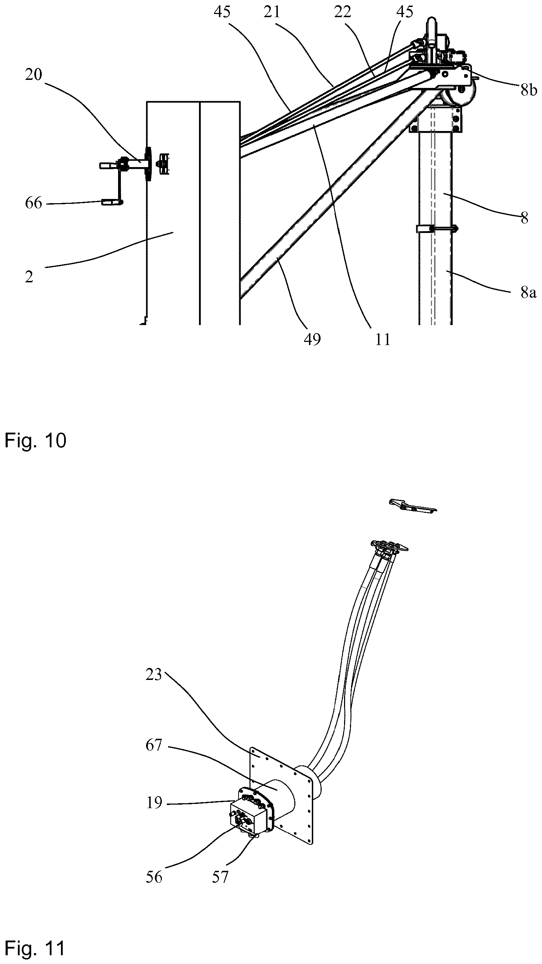

In den Figuren 9 und 10 ist ein weiteres Ausführungsbeispiel eines erfindungsgemäßen Fermenters 1 rein schematisch abgebildet. Dabei entsprechen der Grundaufbau des Fermenters 1 und der Rühreinrichtung 6 im Wesentlichen dem Ausführungsbeispiel gemäß den Figuren 1-3.

Die Führungseinheit 8 mit der daran aufgenommen Rühreinheit 9 wird über eine Konsole 49 an der Fermenterwandung 2 aufgenommen und in Position gehalten. Eine seitliche Abstützung erfolgt über eine Stützeinheit 11.

Bei dem Fermenter 1 in den Figuren 9 und 10 wird die Rühreinheit 9 mechanisch in der Höhe variiert und verschwenkt. Dazu sind zwei Einstelleinrichtungen 13, 14 vorgesehen, die je eine als Stange 45 ausgebildete Einstelleinheit 21, 22 und jeweils eine Bedieneinheit 19, 20 aufweisen.

Die Bedieneinrichtungen 19, 20 weisen jeweils eine Kurbeleinrichtung 47 auf, die hier im gezeigten Ausführungsbeispiel als Handkurbeln 66 ausgebildet sind.

Mit der Bedieneinheit 19 kann die Höhe der Rühreinheit 9 auf dem Stützmast 8a der Führungseinheit 8 eingestellt werden. Mit der Bedieneinheit 20 kann die Rühreinheit 9 in der Höhe verstellt und auch verschwenkt werden.

Die beiden Durchführungseinrichtungen 23, 24 sind als Gelenkdurchführungen 48 ausgebildet, wobei auch nicht näher dargestellte Umlenkeinrichtungen 26 und Koppeleinheiten 27, 28 vorgesehen sind.

Auch in diesem Ausführungsbeispiel ist eine Schutzbügeleinrichtung 45 wenigstens abschnittsweise oberhalb der Führungseinrichtung 8 vorgesehen, um das Foliendach 5 vor eventuellen Beschädigungen zu schützen.

Wie in den Figuren 9 und 10 klar zu sehen ist, steht die Führungseinrichtung 8 nur unwesentlich über die Fermenterwandung 2 nach oben über. Zudem ist keine Serviceöffnung 36 vorgesehen, die z. B. einen Klemmrahmen 53 nötig machen würde.

Daher ist in dem hier gezeigten Ausführungsbeispiel schon ein recht kleiner Schutzbügel 51 ausreichend, der nur über die Stützen 52, 64, 65 an der Führungseinrichtung 8 befestigt ist. Eine Fixierung des Schutzbügels 51 an der Fermenterwandung 2 ist bei dem hier gezeigten Fermenter nicht nötig.

Durch die spezielle Ausgestaltung mit einer sehr flach bauenden Führungseinrichtung 8 und dem rudimentären Schutzbügel 51 können die Anschaffungskosten reduziert werden.

Eine erfindungsgemäße Durchführungseinrichtung 23 für einen Fermenter 1 mit einer hydraulisch höhenverstellbaren und verschwenkbaren Rühreinheit 9 ist in den Figuren 11 bis 13 rein schematisch dargestellt.

Dabei umfasst die Durchführungseinrichtung 23 einen Rohrabschnitt 67, der als Mauerdurchführung 68 durch die Fermenterwandung 2 dient. Zur Fixierung der Durchführungseinrichtung 23 ist eine Befestigungsplatte 69 an dem Rohrabschnitt 27 vorgesehen, mit welcher die Durchführungseinrichtung 23 an der Fermenterwandung 2 z. B. verschraubt wird.

Die Durchführungseinrichtung 23 umfasst in dem hier gezeigten Ausführungsbeispiel auch die Bedieneinheit 19, die eine hydraulische Handpumpe 56 mit einem Hebel 57 und eine Schnittstelle 60 zum Anschluss einer Hydraulikleitung 59 aufweist. Die in den Figuren dargestellten Schläuche stellen die Energieversorgung 55 für die Rühreinheit 9 zur Verfügung. Die Hydraulikschläuche 43, die als Einstelleinheiten 21, 22 der Einstelleinrichtungen 13, 14 zur Ansteuerung des Hydraulikgetriebes 42 dienen, sind in den Figuren nicht näher dargestellt.

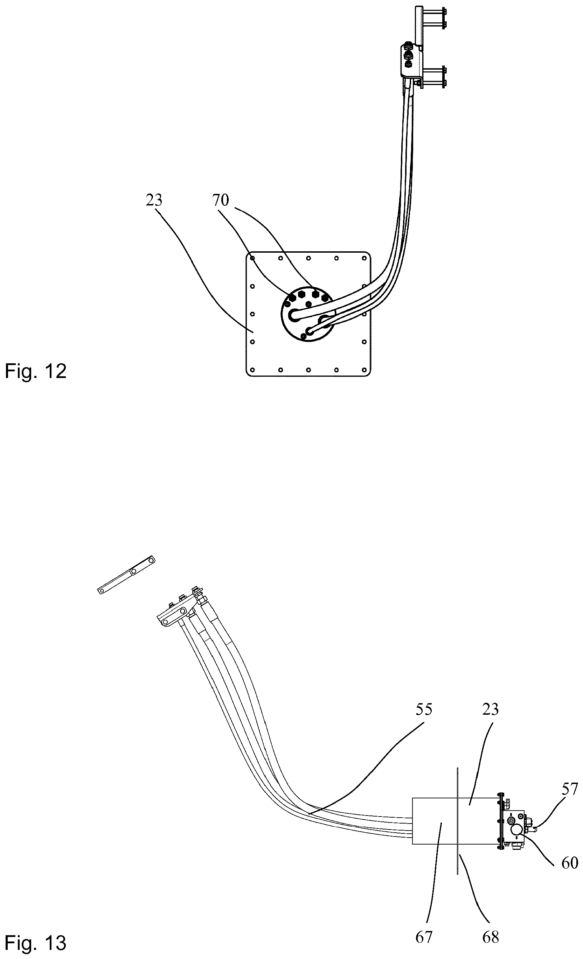

In Figur 12, die eine Rückansicht auf die Durchführungseinrichtung 23 zeigt, sind jedoch die Anschlusseinrichtungen 70 für die zur Verstellung und Verschwenkung notwendigen Hydraulikschläuche 43 zu erkennen.

Insgesamt können die erfindungsgemäßen Rühreinrichtungen 6 mechanisch, hydraulisch und/oder auch pneumatisch betrieben werden. So kann je nach Fermenter 1 und Budget eine passende Rühreinrichtung 6 zusammengestellt werden. Bei den erfindungsgemäßen Rühreinrichtungen 6 können auch verschiedene Betriebsarten kombiniert werden.

**Bezugszeichenliste:**

| | | | |
|---|---|---|---|
| 1 | Fermenter | 36 | Serviceöffnung |
| 2 | Fermenterwandung | 37 | Servicerahmen |
| 3 | Fermenterinnenraum | 38 | Rührflügel |
| 4 | Horizontale | 39 | Drehachse von 8 |
| 5 | Fermenterdach | 40 | Stützgestell |
| 6 | Rühreinrichtung | 41 | maximale Höhe |
| 7 | Substrat | 42 | Hydraulikgetriebe |
| 8 | Führungseinheit | 43 | Hydraulikschlauch |
| 8a | Stützmast | 44 | Hydraulikpumpe |
| 8b | Getriebeeinrichtung | 45 | Schutzbügeleinrichtung |
| 9 | Rühreinheit | 45 | Stange |
| 10 | Biogasanlage | 47 | Kurbeleinrichtung |
| 11 | Stützeinheit | 48 | Gelenkdurchführung |
| 12 | Stützeinheit | 49 | Konsole |
| 13 | Einstelleinrichtung | 50 | Befestigungsanker |
| 14 | Einstelleinrichtung | 51 | Schutzbügel |
| 15 | oberes Ende von 8 | 52 | Stütze |
| 16 | oberes Ende von 2 | 53 | Klemmrahmen |
| 17 | Haltestange | 54 | Querträger |
| 18 | Haltestange | 55 | Energieversorgung |
| 19 | Bedieneinheit | 56 | Handpumpe |
| 20 | Bedieneinheit | 57 | Hebel |
| 21 | Einstelleinheit | 58 | motorisierte Pumpe |
| 22 | Einstelleinheit | 59 | Leitung |
| 23 | Durchführungseinrichtung | 60 | Schnittstelle |
| 24 | Durchführungseinrichtung | 61 | Doppelaufsatzgetriebe |
| 25 | Umlenkeinrichtung | 62 | Getriebemodul |
| 26 | Umlenkeinrichtung | 63 | Getriebemodul |
| 27 | Koppeleinheit | 64 | Stütze |
| 28 | Koppeleinheit | 65 | Stütze |
| 29 | Höhe | 66 | Handkurbel |
| 30 | Schwenkwinkel | 67 | Rohrabschnitt |
| 31 | Winkeleinstelleinrichtung | 68 | Mauerdurchführung |
| | | 69 | Befestigungsplatte |
| 32 | Abstand | 70 | Anschlusseinrichtung |
| 33 | Innenseite | 71 | Befestigungsanker |
| 34 | Außenseite | | |
| 35 | Plattform | | |

## Patentansprüche

1. Fermenter (1) einer Biogasanlage (10) mit wenigstens einer Fermenterwandung (2) und wenigstens einem Fermenterinnenraum (3) und wenigstens einem zur Horizontalen (4) geneigten und insbesondere flexibel ausgebildeten Fermenterdach (5) und mit wenigstens einer Rühreinrichtung (6), um ein in dem Fermenterinnenraum (3) vorhandenes Substrat (7) umzurühren, wobei die Rühreinrichtung (6) wenigstens eine Führungseinheit (8), wenigstens eine Stützeinheit (11, 12) und wenigstens eine an der Führungseinheit (8) aufgenommene verstellbare Rühreinheit (9) aufweist, wobei die Stützeinheit (11, 12) zur Abstützung der Führungseinheit (8) an der Fermenterwandung (2) befestigt ist, wobei die Rühreinheit (9) höhenverstellbar vorgesehen ist, und wobei wenigstens eine Einstelleinrichtung (13, 14) zur Verstellung der Rühreinheit (9) vorgesehen ist,
**dadurch gekennzeichnet,**
**dass** sich das obere Ende (15) der Führungseinheit (8) bis oberhalb eines oberen Endes (16) der Fermenterwandung (2) erstreckt und unterhalb des Fermenterdaches (5) innerhalb des Fermenterinnenraums (3) endet, wobei wenigstens ein Teil der Rühreinheit (9) bis über das obere Ende (16) der Fermenterwandung (2) verstellbar ist und wobei die Einstelleinrichtung (13, 14) wenigstens eine außerhalb des Fermenterinnenraums (3) angeordnete Bedieneinheit (19, 20) und wenigstens eine innerhalb des Fermenterinnenraums (3) angeordnete Einstelleinheit (21, 22) umfasst, die über wenigstens eine gasdichte Durchführungseinrichtung (23, 24) gekoppelt sind und wobei die Bedieneinheit (19, 20) unterhalb des oberen Endes (15) der Führungseinheit (8) und insbesondere auch unterhalb des oberen Endes (16) der Fermenterwandung (2) angeordnet ist und wobei in dem Fermenterdach (5) wenigstens eine gasdicht verschließbare Serviceöffnung (36) oberhalb der Führungseinheit (8) vorgesehen ist.

2. Fermenter nach Anspruch 1, wobei die Drehachse (17) der Rühreinheit (9) bis über das obere Ende (16) der Fermenterwandung (2) verstellbar ist und/oder wobei die Rühreinheit (9) vollständig bis oberhalb des oberen Endes (16) der Fermenterwandung (2) verstellbar ist.

3. Fermenter nach einem der vorhergehenden Ansprüche, wobei die Stützeinheit (11, 12) wenigstens eine sich von der Fermenterwandung (2) aus schräg nach oben erstreckende Haltestange (17, 18) umfasst.

4. Fermenter nach einem der vorhergehenden Ansprüche, wobei die Führungseinheit (8) wenigstens einen Stützmast (8a) und wenigstens eine Getriebeeinrichtung (8b) umfasst und/oder wobei die Einstelleinheit (21, 22) mit der Getriebeeinrichtung (8b) in Wirkverbindung steht und/oder wobei die Einstelleinheit (21, 22) mechanisch, hydraulisch und/oder pneumatisch ausgebildet ist.

5. Fermenter nach einem der vorhergehenden Ansprüche, wobei die Getriebeeinrichtung (8b) als Hydraulikgetriebe (42) ausgebildet ist und die Einstelleinheit (21, 22) wenigstens einen Hydraulikschlauch (43) umfasst oder als ein solcher ausgebildet ist, wobei der Hydraulikschlauch (43) das Hydraulikgetriebe (42) mit der Bedieneinheit (19, 20) verbindet, welche wenigstens eine Hydraulikpumpe (44) umfasst.

6. Fermenter nach einem der Ansprüche 1-5 wobei die Einstelleinrichtung (13, 14) mechanisch ausgebildet, wobei die Einstelleinheiten (21, 22) als Stangen (45) ausgebildet sind und wobei die Bedieneinheit (19, 20) wenigstens eine Kurbeleinrichtung umfasst.

7. Fermenter nach dem vorhergehenden Anspruch, wobei die gasdichte Durchführungseinrichtung (23, 24) unterhalb des Fermenterdaches (5) und insbesondere in der Fermenterwandung (2) angeordnet ist.

8. Fermenter nach einem der vorhergehenden Ansprüche, wobei die Einstelleinheit (21, 22) wenigstens eine Umlenkeinrichtung umfasst, wobei die Umlenkeinrichtung insbesondere wenigstens eine Kardangelenkeinrichtung (25, 26) und/oder wenigstens eine Winkelantriebseinrichtung und/oder wenigstens eine biegsame Welle umfasst oder als eine solche ausgebildet ist.

9. Fermenter nach einem der vorhergehenden Ansprüche, wobei die Einstelleinheit (21, 22) eine zur Horizontalen (4) geneigte Koppeleinheit (27, 28) umfasst und wobei insbesondere die Umlenkeinrichtung (25, 26) zwischen der Koppeleinheit (27, 28) und der Durchführungseinrichtung (23, 24) angeordnet ist.

10. Fermenter nach einem der beiden vorhergehenden Ansprüche, wobei die Rühreinheit (9) verschwenkbar vorgesehen ist und wobei der Schwenkwinkel (30) über eine Winkeleinstelleinrichtung (31) einstellbar ist und/oder wobei ein Abstand (32) der Führungseinheit (8) von der Innenseite (33) der Fermenterwandung (2) so bemessen ist, dass die Rühreinheit (9) zur Fermenterwandung (2) hin verschwenkbar ist.

11. Fermenter nach einem der vorhergehenden Ansprüche, wobei wenigstens eine Plattform (35) an der Fermenterwandung (2) und/oder der Stützeinheit (11, 12) vorgesehen und insbesondere befestigt ist und/oder wobei die Serviceöffnung (36) in einem Servicerahmen (37) vorgesehen ist und wobei insbesondere an dem Servicerahmen (37) wenigstens eine gasdichte Durchführungseinrichtung (23, 24) vorgesehen ist.

12. Fermenter nach einem der vorhergehenden Ansprüche, wobei wenigstens eine Schutzbügeleinrichtung (45) unterhalb des Fermenterdaches vorgesehen ist und wobei insbesondere der Servicerahmen (37) an der Schutzbügeleinrichtung (45) aufgenommen ist.

13. Fermenter nach einem der vorhergehenden Ansprüche, wobei die Einstelleinrichtung (13, 14) händisch und/oder motorisiert betreibbar ist.

## Claims

1. Fermenter (1) of a biogas plant (10) comprising at least one fermenter wall (2) and at least one fermenter interior (3) and at least one fermenter roof (5) which is inclined towards the horizontal (4) and in particular is configured to be flexible and comprising at least one stirring device (6) in order to stir a substrate (7) present in the fermenter interior (3), wherein the stirring device (6) comprises at least one guide unit (8), at least one support unit (11, 12) and at least one adjustable stirring unit (9) received on the guide unit (8), wherein the support unit (11, 12) is fastened to the fermenter wall (2) to support the guide unit (8), wherein the stirring unit (9) is provided to be height-adjustable and wherein at least one adjusting device (13, 14) is provided for adjusting the stirring unit (9), **characterized in that** the upper end (15) of the guide unit (8) extends as far as above an upper end (16) of the fermenter wall (2) and ends below the fermenter roof (5) inside the fermenter interior (3), wherein at least one part of the stirring unit (9) is adjustable as far as above the upper end (16) of the fermenter wall (2) and wherein the adjusting device (13, 14) comprises at least one operating unit (19, 20) arranged outside the fermenter interior (3) and at least one adjusting unit (21, 22) arranged inside the fermenter interior (3) which are coupled by means of at least one gastight feed-through device (23, 24) and wherein the operating unit (19, 20) is arranged underneath the upper end (15) of the guide unit (8) and in particular also underneath the upper end (16) of the fermenter wall (2) and wherein in the fermenter roof (5) at least one gastight closable service opening (36) is provided above the guide unit (8).

2. Fermenter according to claim 1, wherein the axis of rotation (17) of the stirring unit (9) is adjustable as far as above the upper end (16) of the fermenter wall (2) and/or wherein the stirring unit (9) is adjustable completely as far as above the upper end (16) of the fermenter wall (2).

3. Fermenter according to one of the preceding claims, wherein the support unit (11, 12) comprises at least one retaining rod (17, 18) extending obliquely upwards out from the fermenter wall (2).

4. Fermenter according to one of the preceding claims, wherein the guide unit (8) comprises at least one support mast (8a) and at least one transmission device (8b) and/or wherein the adjusting unit (21, 22) is in operative communication with the transmission device (8b) and/or wherein the adjusting unit (21, 22) is configured to be mechanical, hydraulic and/or pneumatic.

5. Fermenter according to one of the preceding claims, wherein the transmission device (8b) is configured as a hydraulic transmission (42) and the adjusting unit (21, 22) comprises at least one hydraulic hose (43) or is configured as such, wherein the hydraulic hose (43) connects the hydraulic transmission (42) to the operating unit (19, 20) which comprises at least one hydraulic pump (44) .

6. Fermenter according to one of Claims 1-5, wherein the adjusting device (13, 14) is configured to be mechanical, wherein the adjusting units (21, 22) are configured as rods (45) and wherein the operating unit (19, 20) comprises at least one crank device.

7. Fermenter according to the preceding claim, wherein the gastight feed-through device (23, 24) is arranged underneath the fermenter roof (5) and in particular in the fermenter wall (2).

8. Fermenter according to one of the preceding claims, wherein the adjusting unit (21, 22) comprises at least one deflecting device, wherein the deflecting device in particular comprises at least one cardan joint device (25, 26) and/or at least one angle drive device and/or at least one flexible shaft or is configured as such.

9. Fermenter according to one of the preceding claims, wherein the adjusting unit (21, 22) comprises a coupling unit (27, 28) inclined towards the horizontal (4) and wherein in particular the deflecting device (25, 26) is arranged between the coupling unit (27, 28) and the feed-through device (23, 24).

10. Fermenter according to one of the two preceding claims, wherein the stirring unit (9) is provided to be pivotable and wherein the pivot angle (30) is adjustable by means of an angle adjusting device (31) and/or wherein a distance (32) of the guide unit (8) from the inner side (33) of the fermenter wall (2) is dimensioned so that the stirring unit (9) is pivotable towards the fermenter wall (2).

11. Fermenter according to one of the preceding claims, wherein at least one platform (35) is provided and in particular is fastened on the fermenter wall (2) and/or support unit (11, 12) and/or wherein the service opening (36) is provided in a service frame (37) and wherein in particular at least one gastight feed-through device (23, 24) is provided on the service frame (37).

12. Fermenter according to one of the preceding claims, wherein at least one protective bracket device (45) is provided underneath the fermenter roof and wherein in particular the service frame (37) is received at the protective bracket device (45).

13. Fermenter according to one of the preceding claims, wherein the adjusting device (13, 14) can be operated by hand and/or in a motorized manner.

## Revendications

1. Digesteur (1) d'une installation de biogaz (10), pourvu d'au moins une paroi de digesteur (2) et d'au moins un espace intérieur de digesteur (3) et d'au moins un toit de digesteur (5) incliné vers l'horizontale (4) et conçu notamment en version flexible, et pourvu d'au moins un système agitateur (6), destiné à brasser un substrat (7) présent dans l'espace intérieur de digesteur (3), le système agitateur (6) comportant au moins une unité de guidage (8), au moins une unité de support (11, 12) et au moins une unité agitatrice (9) ajustable, réceptionnée sur l'unité de guidage (8), pour soutenir l'unité de guidage (8), l'unité de support (11, 12) étant fixée sur la paroi de digesteur (2), l'unité agitatrice (9) étant prévue en étant ajustable en hauteur, et au moins un système de réglage (13, 14) étant prévu pour ajuster l'unité agitatrice (9),
**caractérisé**
**en ce que** l'extrémité supérieure (15) de l'unité de guidage (8) s'étend jusqu'au-dessus d'une extrémité supérieure (16) de la paroi de digesteur (2) et se termine en-dessous du toit de digesteur (5), à l'intérieur de l'espace intérieur de digesteur (3), au moins une partie de l'unité agitatrice (9) étant ajustable jusqu'au-delà de l'extrémité supérieure (16) de la paroi de digesteur (2) et le système de réglage (13, 14) comprenant au moins une unité de commande (19, 20) placée à l'extérieur de l'espace intérieur de digesteur (3) et au moins une unité de réglage (21, 22) placée à l'intérieur de l'espace intérieur de digesteur (3) qui sont accouplées par l'intermédiaire d'au moins un système de passage (23, 24) étanche au gaz et l'unité de commande (19, 20) étant placée en-dessous de l'extrémité supérieure (15) de l'unité de guidage (8) et notamment aussi en-dessous de l'extrémité supérieure (16) de la paroi de digesteur (2) et dans le toit de digesteur (5), au moins une ouverture de regard (36) fermable de manière étanche au gaz étant prévue au-dessus de l'unité de guidage (8).

2. Digesteur selon la revendication 1, l'axe de rotation (17) de l'unité agitatrice (9) étant ajustable jusqu'au-delà de l'extrémité supérieure (16) de la paroi de digesteur (2) et/ou l'unité agitatrice (9) étant entièrement ajustable jusqu'au-dessus de l'extrémité supérieure (16) de la paroi de digesteur (2).

3. Digesteur selon l'une quelconque des revendications précédentes, l'unité de support (11, 12) comprenant au moins une barre de maintien (17, 18) s'étendant en oblique vers le haut à partir de la paroi de digesteur (2).

4. Digesteur selon l'une quelconque des revendications précédentes, l'unité de guidage (8) comprenant au moins un mât de soutien (8a) et au moins un système de transmission (8b) et/ou l'unité de réglage (21, 22) étant en liaison active avec le système de transmission (8b) et/ou l'unité de réglage (21, 22) étant conçue en version mécanique, hydraulique et/ou pneumatique.

5. Digesteur selon l'une quelconque des revendications précédentes, le système de transmission (8b) étant conçu sous la forme d'une transmission hydraulique (42) et l'unité de réglage (21, 22) comprenant au moins un flexible hydraulique (43) ou étant conçue en tant que tel, le flexible hydraulique (43) reliant la transmission hydraulique (42) avec l'unité de commande (19, 20), laquelle comprend au moins une pompe hydraulique (44).

6. Digesteur selon l'une quelconque des revendications 1 à 5, le système de réglage (13, 14) étant conçu en version mécanique, les unités de réglage (21, 22) étant conçues sous la forme de barres (45) et l'unité de commande (19, 20) comprenant au moins un système de manivelle.

7. Digesteur selon la revendication précédente, le système de passage (23, 24) étanche au gaz étant placé en-dessous du toit de digesteur (5) et notamment dans la paroi de digesteur (2).

8. Digesteur selon l'une quelconque des revendications précédentes, l'unité de réglage (21, 22) comprenant au moins un système de renvoi, le système de renvoi comprenant notamment au moins un système de joint de cardan (25, 26) et/ou au moins un système d'entraînement angulaire et/ou au moins un arbre flexible ou étant conçu en tant que tel.

9. Digesteur selon l'une quelconque des revendications précédentes, l'unité de réglage (21, 22) comprenant une unité de couplage (27, 28) inclinée ver l'horizontale (4) et notamment le système de renvoi (25, 26) étant placé entre l'unité de couplage (27, 28) et le système de passage (23, 24).

10. Digesteur selon l'une quelconque des deux revendications précédentes, l'unité agitatrice (9) étant prévue en étant apte à pivoter et l'angle de pivotement (30) étant réglable par l'intermédiaire d'un système de réglage angulaire (31) et/ou un écart (32) de l'unité de guidage (8) par rapport à la face interne (33) de la paroi de digesteur (2) étant dimensionné de telle sorte que l'unité agitatrice (9) soit apte à pivoter vers la paroi de digesteur (2).

11. Digesteur selon l'une quelconque des revendications précédentes, au moins une plateforme (35) étant prévue et notamment fixée sur la paroi de digesteur (2) et/ou sur l'unité de support (11, 12) et/ou l'ouverture de regard (36) étant prévue dans un cadre de regard (37) et notamment sur le cadre de regard (37) étant prévu au moins un système de passage (23, 24) étanche au gaz.

12. Digesteur selon l'une quelconque des revendications précédentes, au moins un système d'étrier protecteur (45) étant prévu en-dessous du toit de digesteur et notamment le cadre de regard (37) étant réceptionné sur le système d'étrier protecteur (45).

13. Digesteur selon l'une quelconque des revendications précédentes, le système de réglage (13, 14) étant actionnable manuelle et/ou par moteur.
